(19) Europäisches Patentamt

European Patent Office

Office européen des brevets

(11) **EP 2 310 968 B1**

(12) **EUROPEAN PATENT SPECIFICATION**

(45) Date of publication and mention of the grant of the patent:
**23.08.2017 Bulletin 2017/34**

(21) Application number: **09766259.7**

(22) Date of filing: **12.06.2009**

(51) Int Cl.:
*G06F 19/22* (2011.01)       *G06F 19/24* (2011.01)
*G06F 19/26* (2011.01)

(86) International application number:
**PCT/IB2009/052517**

(87) International publication number:
**WO 2009/153722 (23.12.2009 Gazette 2009/52)**

(54) **A METHOD FOR SPECTRAL DNA ANALYSIS**

VERFAHREN FÜR DNA-SPEKTRALANALYSE

PROCÉDÉ D'ANALYSE SPECTRALE D'ADN

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **19.06.2008 EP 08158610**

(43) Date of publication of application:
**20.04.2011 Bulletin 2011/16**

(73) Proprietor: **Koninklijke Philips N.V.**
**5656 AE Eindhoven (NL)**

(72) Inventors:
• **BUCUR, Anca, I., D.**
**NL-5656 AE Eindhoven (NL)**
• **VAN LEEUWEN, Jasper, J., A.**
**NL-5656 AE Eindhoven (NL)**
• **DIMITROVA, Nevenka**
**NL-5656 AE Eindhoven (NL)**
• **MITTAL, Chetan**
**NL-5656 AE Eindhoven (NL)**

(74) Representative: **Kroeze, Johannes Antonius**
**Philips Intellectual Property & Standards**
**High Tech Campus 5**
**5656 AE Eindhoven (NL)**

(56) References cited:
• SUSSILLO D ET AL: "Spectrogram analysis of genomes" EURASIP JOURNAL OF APPLIED SIGNAL PROCESSING, HINDAWI PUBLISHING CO., CUYAHOGA FALLS, OH, US, vol. 2004, no. 1, 1 January 2004 (2004-01-01), pages 29-42, XP002452661 ISSN: 1110-8657
• RENJUN Y ET AL: "Spectrogram analysis of genome small patterns using pseudo smoothed Wigner-Ville distribution" 5TH INTERNATIONAL CONFERENCE ON INFORMATION COMMUNICATIONS AND SIGNAL PROCESSING, IEEE, US, 1 January 2005 (2005-01-01), pages 1044-1047, XP002498578 ISBN: 978-0-7803-9282-3
• SHARMA DEEPAK ET AL: "Spectral Repeat Finder (SRF): identification of repetitive sequences using Fourier transformation" BIOINFORMATICS, OXFORD UNIVERSITY PRESS, SURREY, GB, vol. 20, no. 9, 12 June 2004 (2004-06-12), pages 1405-1412, XP002493846 ISSN: 1367-4803
• ANASTASSIOU DIMITRIS: "Frequency-domain analysis of biomolecular sequences" BIOINFORMATICS, OXFORD UNIVERSITY PRESS, SURREY, GB, vol. 16, no. 12, 1 December 2000 (2000-12-01), pages 1073-1081, XP002498577 ISSN: 1367-4803

EP 2 310 968 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to a method for performing spectral DNA analysis, i.e. DNA sequences being represented in spectral space using Fourier transform. The invention also relates to a corresponding computer program product.

BACKGROUND OF THE INVENTION

DNA Spectrogram method from DNA sequence has been described in the past, cf. Benson et al. in Nucleic Acid Research. 18 (21), p. 6305-6310, and 18 (10), 3001-3006, 1990, for earlier references on this topic.

A DNA spectrogram is generated by converting a DNA sequence to binary indicator sequence and then applying short-time Fourier transform and mapping to a color space in order to visualize the output. In order to allow the phylogenetic and biological comparison of a large number of long sequences in frequency domain, these sequences need to be visualized in such a way that the similarities are (easily) detectable, even by a human observer. Therefore, strategies are required that group together sequences with similar patterns in frequency.

An important advantage of performing DNA analysis in the spectral domain is that the $N^2$-scaling of conventional sequence to sequence matching is avoided, N being the number nucleotide bases in the sequence. US 6,287,773 discloses e.g. a frequency domain based comparison method which scale as N log (N) which may very significantly lower the computational time for long sequences, e.g. longer than 10.000 nucleotide bases.

Even with the advantages of present spectral analysis for DNA analysis, there is still a need for even faster and/or more efficient analysis tools because of the huge amount of data. For example, the entire chromosome 1 of the human genome is 247 millions nucleotides long, and accordingly viewing the DNA spectrograms as so-called spectra video as recently suggested by N. Dimitrova et al., "Analysis and visualization of DNA spectrograms: open possibilities for genome research," in ACM MM., Santa Barbara, CA, Oct. 2006, may also be a tedious task.

Moreover, despite efforts to date, a need remains for systems and methods that facilitate expeditious analysis of DNA sequence information. Also there remains a need for tools that can identify structurally or compositionally similar patterns that exhibit similar spectral properties. Such tools are to be contrasted with conventional sequence alignment tools that seek to align sequences in linear order or by nucleotide appearance.

Clustering algorithms currently used for sequence alignment are not suitable for spectral analysis, where we need to analyze the content at individual frequencies. Standard clustering methods involve a global distance metric, which in this case would be applied over all frequencies considered in the spectrogram. While such a method would be able to detect strong patterns in many frequencies, it would screen out strong patterns in individual frequencies. However, there is no relation between patterns on different frequencies to consider them in a single distance metric. In spectral analysis strong (long) patterns on single frequencies are relevant.

Hence, an improved method for analysis of DNA sequences would be advantageous, and in particular a more efficient and/or reliable method would be advantageous.

SUMMARY OF THE INVENTION

Accordingly, the invention preferably seeks to mitigate, alleviate or eliminate one or more of the above mentioned disadvantages singly or in any combination. In particular, it may be seen as an object of the present invention to provide a method that solves the above mentioned problems of the prior art with analysis of DNA sequences.

This object and several other objects are obtained in a first aspect of the invention by providing a method for identifying structurally or compositionally similar patterns of a DNA sequence that exhibit similar spectral properties, the method comprising:

- providing a DNA sequence,
- creating a plurality of spectra based on the DNA sequence by converting the DNA sequence into a plurality of binary indicator sequences (BIS), and applying short term Fourier transform (STFT) on the binary indicator sequences, each spectrum comprising corresponding frequencies (k) and Fourier coefficients (Usk_X(k)), where each kind of Fourier coefficient constitutes a channel (X),
- defining a binning function (BF) for a frequency (K') applicable to the Fourier coefficients (Usk_X(k)) with respect to one or more channels (X),
- applying the binning function (BF) on at least a portion of the plurality of spectra and thereby modifying the corresponding Fourier coefficients (Usk_X(k)), and
- finding substantially equal modified Fourier coefficients (Usk_X(k)) within the said portion of the plurality of spectra, and

wherein a first group of spectra (S) with the largest set of substantially equal modified Fourier coefficients (Usk_X(k)) in any frequency and/or channel is found and separated from the remaining spectra, the remaining spectra forming a second group of spectra.

The invention is particularly, but not exclusively, advantageous for obtaining a method for providing a user with a much improved ability to see unique strong patterns in vast amount of DNA sequence data. It is further possible to extracting the strength of a pattern and evaluate which is the strongest pattern on an individual frequency or a set of frequencies, or all patterns on all frequencies in the DNA sequences to analyze.

The invention may beneficially be implemented with a fully or semi-automated pattern search through all the DNA spectra coupled with an annotation and/or a visualization environment.

The use of binning functions (BF) may allow for a flexible measure of "similarity" which can be adapted to the dataset in order to detect all relevant patterns, coping with variation in the DNA sequences.

Additionally, the invention is scalable and suitable for parallel implementation that makes feasible search through vast genomic data spaces such as genomes of different species.

This method may efficiently and effectively compare multiple large genomic sequences based on their spectral patterns for deriving the gene homology and hence phylogenetic relationships

A common spectral pattern among sequences could for instance identify periodic repeating of nucleotides in the sequences and would help discover novel repeat elements in coding and non-coding DNA which may not otherwise be "visible" due to periodicity of only specific nucleotides after randomly arranged nucleotides in the periodic interval.

In the context of the present invention, other method for spectral analysis may also be beneficially applied, as for example the method described in PCT Application PH008112WO1 (Attorney Reference), IB2008/051434 (PCT Application number).

The binning function may comprise truncation, rounding up, rounding down, a modular function, and/or a threshold function or any other relevant binning function available to the skilled person that may implemented in connection with the present invention.

said binning function (BF). Thus, it may comprise plotting the said distribution, e.g. plotting in a histogram, as it will be explained in more detail below, or other kind of graphs.

Typically, the method is repeated for a set of frequencies (K_i), e.g. all frequencies, or an interval, continuously or not continuously i.e. disjoint, depending on the requirements of the desired analysis.

It should be noted, the method may equally be applied for analyzing a RNA sequence or an amino acid sequence instead of a DNA sequence. The application of the present invention is thus not limited to applications in connection with analysis of DNA sequences, but could also be applied on similar sequences of relevance within biochemistry e.g. RNA sequences and amino acid sequences.

One may create a binary indicator representation for amino acids (20 of them) and then we apply STFT to convert the BIS sequences to Fourier domain space. Then the rest of the procedure for implementing the invention would be the same. Here is a list of the aminoacids:

    alanine - ala - A
    arginine - arg - R
    asparagine - asn - N
    aspartic acid - asp - D
    cysteine - cys - C
    glutamine - gln - Q
    glutamic acid - glu - E
    glycine - gly - G
    histidine - his - H
    isoleucine - ile - I
    leucine - leu - L
    lysine - lys - K
    methionine - met - M
    phenylalanine - phe - F
    proline - pro - P
    serine - ser - S
    threonine - thr - T
    tryptophan - trp - W
    tyrosine - tyr - Y
    valine - val - V

proline - pro - P
serine - ser - S
threonine - thr - T
tryptophan - trp - W
tyrosine - tyr - Y
valine - val - V

The 20 different amino acids can be mapped to 20 different colors in Red-Green-Blue (RGB) (or Hue Saturation Value - HSV space). Either one of these spaces can be quantized into 20 colors - one for each of the amino acids. Thus, the teaching of the present invention is not limited to DNA analysis, but may be extended to RNA and/or amino acid analysis with the relevant modifications readily recognized by a skilled person in this field.

Preferably, the set of binary indicator sequences may be reduced into a smaller set of BIS using a merge function, the merge function may preferably comprise a logical AND function.

The set of found substantially equal modified Fourier coefficients (Usk_X(k)) within the said portion of the plurality of spectra may be defined to constitute a pattern. The term "largest set" means collective group with the highest number of re-occurring modified Fourier coefficients. Additionally, the largest set of substantially equal modified Fourier coefficients (Usk_X(k)) may be found and separated within the second group of spectra. Moreover, the separation of spectra into a first and a second group of spectra may be repeated disregarding the previously found longest set of modified Fourier coefficients (Usk_X(k)), thus finding next longest. The separation of spectra into a first and a second group may be repeated i) until a pre-defined threshold for the longest set of modified Fourier coefficients (Usk_X(k)) is found, ii) until a pre-defined number of separations into a first and a second group of spectra is performed, or iii) until the first and/or the second group of spectra contains a single sequence, so as to provide an end to the separation.

In one embodiment, a first group of spectra (S) with the largest set of substantially equal modified Fourier coefficients (Usk_X(k)) in any frequency and/or channel may be found and marked. The set may preferably be displayed for analysis. Furthermore, a second group of spectra with the largest set of substantially equal modified Fourier coefficients (Usk_X(k)) in any frequency and/or channel may be found, disregarding the (Usk_X(k)) is found, ii) until a pre-defined number of longest set is found, or iii) until the longest set contains a single sequence, so as to provide an end to the process of this embodiment.

In another embodiment, all groups of spectra (S) having length of a pattern of found modified Fourier coefficients (Usk_X(k)) being above a first predefined threshold (N_thres1), or all groups of spectra containing the k longest patterns, k being an integer, may be found and separated from the remaining spectra, the remaining spectra forming a second group of spectra. The groups of spectra selected are not necessarily disjoint. Each group of spectra thus separated may be further separated using a second predefined threshold (N_thres2) for the length of the pattern of modified Fourier coefficients (Usk_X(k)), or using the **j** longest patterns, **j** being an integer equal or different from **k**. To provide an end to the separation, the separation of spectra into groups may be repeated i) until a pre-defined threshold for length of the patterns of modified Fourier coefficients (Usk_X(k)) is found, ii) until a pre-defined number of separations into a first and a second group of spectra is performed, or iii) until the first and/or the second group of spectra contains sequences of modified Fourier coefficients (Usk_X(k)) having length equal to one.

In a second aspect, the invention relates to a computer program product being adapted to enable a computer system comprising at least one computer to implement the method according to the first aspect of the invention.

This aspect of the invention is particularly, but not exclusively, advantageous in that the present invention may be implemented by a computer program product enabling a computer system to perform the operations of the second aspect of the invention. Thus, it is contemplated that some known computer system may be changed to operate according to the present invention by installing a computer program product on a computer system controlling the said optical recording apparatus. Such a computer program product may be provided on any kind of computer readable medium, e.g. magnetically or optically based medium, or through a computer based network, e.g. the Internet.

The invention can be implemented in any suitable form including hardware, software, firmware or any combination of these. The invention or some features of the invention can be implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

These and other aspects of the invention will be apparent from and elucidated with reference to the embodiments described hereinafter.

BRIEF DESCRIPTION OF THE FIGURES

The present invention will now be explained, by way of example only, with reference to the accompanying Figures, where

Fig. 1 is an exemplary binary sequence (BIS) pattern,
Fig. 2 is a plot of the corresponding BIS pattern from Figure 1 of the four nucleotide bases A, T, C, and G,
Fig. 3 is the converted frequency spectrum of each base,
Fig. 4 is similar to Figure 3 and it is indicated to the right that a superposition of the color mapping vectors weighted by the magnitude of the frequency component of the respective nucleotide base is obtained,
Fig. 5 schematically illustrates the generation of a single, colored spectrum from short time Fourier transformation (STFT) of a part of a DNA sequence,
Fig. 6 is similar to Figure 5, and illustrates the generation of a plurality of spectra by repeated STFT along the DNA sequence,
Fig. 7 is a principle sketch of the application of a binning function (BF) according to the present invention,
Fig. 8 is a schematic drawing of the spectra at various frequencies according to the present invention,
Fig. 9 is a drawing similar to Figure 8 showing a binning function (BF) according to the present invention,
Fig. 10 is a drawing similar to Figure 8 showing another binning function (BF') according to the present invention,
Fig. 11 is a drawing similar to Figure 8 schematically illustrating application of a binning function and plotting into a histogram according to the present invention,
Fig. 12 and 13 show an example of a so-called top-down hierarchical sorting (TDHS) according to the present invention,
Fig. 14 and 15 show an example of a so-called independent iterative sorting (IIS) according to the present invention, and
Fig. 16 is a flow chart of a method according to the invention.

## DETAILED DESCRIPTION OF AN EMBODIMENT

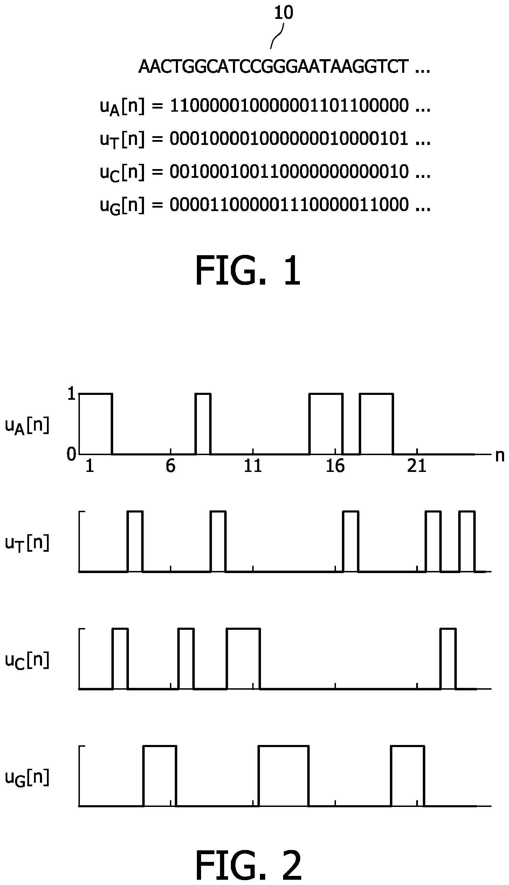

DNA spectrograms can be generated in a conventional manner, as described in greater detail herein below with reference to Fig. 1-6. For example, a conventional algorithm or technique for the generation of DNA spectrograms may be employed that entails the following five steps:

(i) *Formation of binary indicator sequences (BISs) $u_A[n]$, $u_T[n]$, $u_C[n]$ and $u_G[n]$ for the four nucleotide bases.* An exemplary BIS pattern is reproduced in Fig. 1 generated from a DNA sequence 10 and a plot of the BIS values is presented in Figure. 2.

(ii) *Discrete Fourier Transform (DFT) on BISs.* The frequency spectrum of each base is obtained by computing the DFT of its corresponding BIS using Equation (1):

$$U_X[k] = \sum_{n=0}^{N-1} u_X[n] e^{-j\frac{2\pi}{N}kn} \ , \ k = 0, 1, ..., \lfloor N/2 \rfloor + 1 \quad X = A, T, C \ or \ G \qquad (1)$$

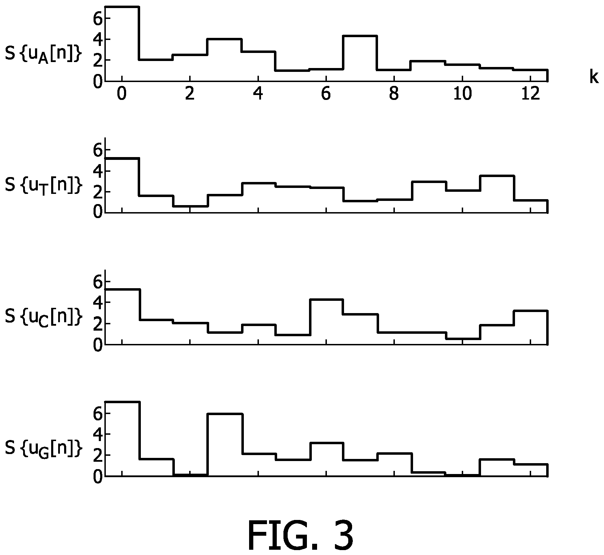

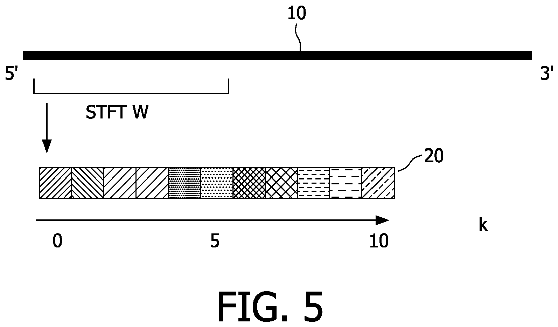

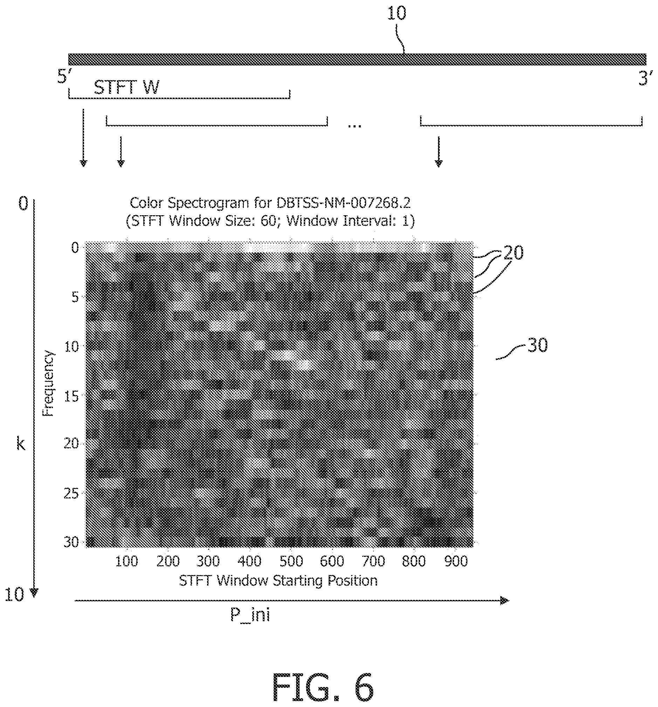

As illustrated in Figure 3, the sequence $U[k]$ provides a measure of the frequency content at frequency k, which is equivalent to an underlying period of N/k samples. N is the total number of N is the total number of nucleotide bases in the window W, cf. Figures 5 and 6. The number of bases can be maximum 300 nucleotide bases, preferably maximum 500 nucleotide bases, or even more preferably 700 nucleotide bases. Alternatively, the period can be maximum 3000 nucleotide bases, preferably maximum 5000 nucleotide bases, or even more preferably maximum 10000 nucleotide bases..

(iii) *Mapping of DTF values to RGB colors.* The four DFT sequences are reduced to three sequences in the RGB space by a set of linear equations which are reproduced below:

$$X_r[k] = a_r U_A[k] + t_r U_T[k] + c_r U_C[k] + g_r U_G[k]$$
$$X_g[k] = a_g U_A[k] + t_g U_T[k] + c_g U_C[k] + g_g U_G[k] \quad (2)$$
$$X_b[k] = a_b U_A[k] + t_b U_T[k] + c_b U_C[k] + g_b U_G[k]$$

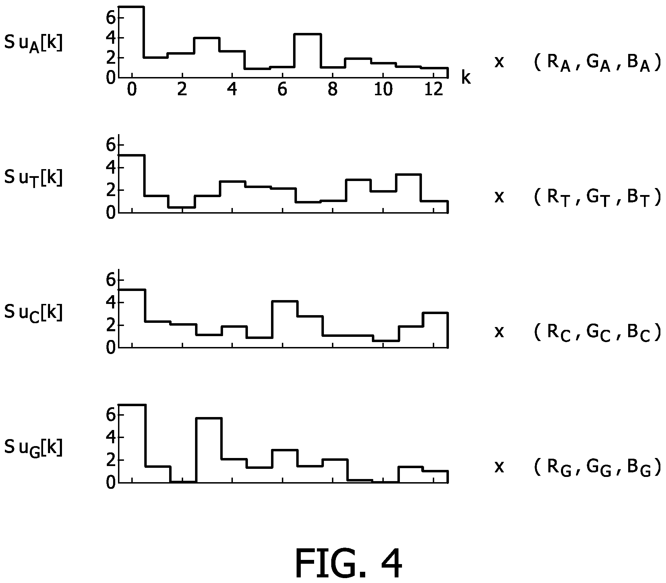

where $(a_r, a_g, a_b)$, $(t_r, t_g, t_b)$, $(c_r, c_g, c_b)$ and $(g_r, g_g, g_b)$ are the colour mapping vectors for the nucleotide bases A, T, C and G, respectively. The resultant pixel color $((X_r[k], X_g[k], X_b[k])$ is thus a superposition of the colour mapping vectors weighted by the magnitude of the frequency component of their respective nucleotide base as indicated in the right side of Figure 4. Mapping of DFT values to colors is illustrated in Figure 5 for a single spectrum 20, and in Figure 6 for several spectra 20 i.e. a spectrogram 30. Both Figures 5 and 6 are being reproduced in grey-tones here for illustrative purposes. Other color space mapping of the frequency domain based U values are also possible, e.g. to HSV space.

(iv) *Formalizing the pixel values.* Before rendering the colored spectrogram 30, the RGB values of each pixel are generally normalized so as to fall between 0 and 1. Numerous normalization procedures are readily available to the skilled person once the general principle of the invention is recognized.

(v) *Short-time Fourier Transform (STFT).* A plurality of DNA spectra 20 i.e. a spectrogram 30 is formed by a concatenation of individual DNA sequence spectra 20 ("strips"), where each strip or spectrum generally depicts the frequency spectrum of a local DNA segment as shown in Figure 6. The short term Fourier transform (STFT) has a window W that is shifted along the DNA sequence from 5' to 3' as shown in Figure 6.

The spectrogram shown in Figure 6 has a length of 60 nucleotide bases and the window W is shifted one base at a time. On the horizontal scale in the spectrogram 30, the frequency k is shown (increasing downwards), whereas the start position P_ini on the DNA sequence 10 is shown on the horizontal scale in the spectrogram 30.

The appearance of a spectrogram 30 is very much affected by the choice of the STFT window W size, the length of the overlapping sequence between adjacent windows W, and the color mapping vectors, cf. Equation (2). The window size determines the effective range of a pixel value in a spectrogram 30. A larger window results in a spectrogram that reveals statistics collected from longer DNA segments. In general, the window W size should be made several times larger than the length of the repetitive pattern of interest and smaller than the size of the region that contain the pattern of interest. For exploratory purposes, it is recommended to try a range of window sizes. The window overlap determines the length of the DNA segment common to two adjacent STFT windows. Therefore, the larger the overlap, the more gradual is the transition of the frequency spectrum from one STFT window to the next. A higher image resolution makes it easier to extract features by image processing or visual inspection.

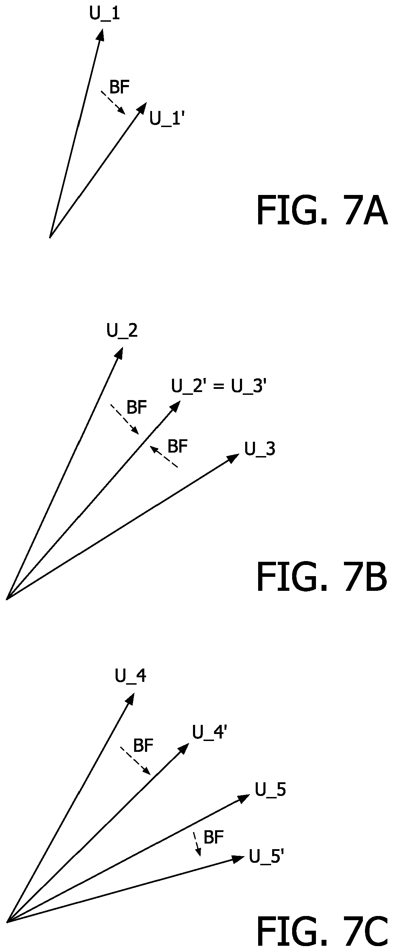

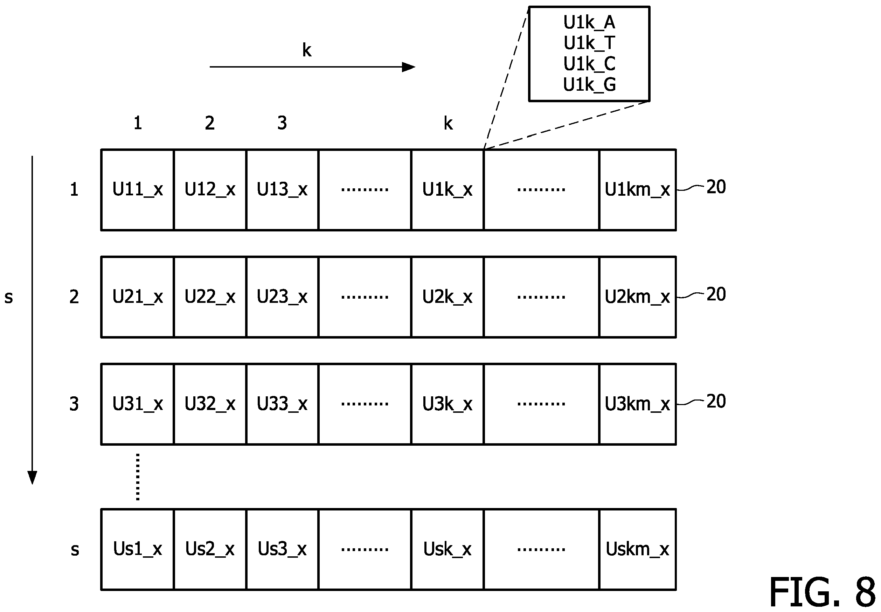

Viewing large amounts of sequence data requires an efficient method for information analysis and visualization. In order to optimize the viewing of spectra derived from very large sequences or spectra containing many small windows, the spectra can be rendered a video as shown by the present inventor; N. Dimitrova, et al. "Analysis and visualization of DNA spectrograms: open possibilities for genome research," in ACM MM., Santa Barbara, CA, Oct. 2006. Fig. 7 is a principle sketch of the application a binning function according to three different situations according to the present invention. With reference to Figure 3 and 8 (cf. below), the four channels A, T, C, and G, each defining a three-dimensional space in reciprocal k-space by the coordinates frequency k, Fourier coefficient Usk_X(k), and the spectra number s. Thus, for one channel a frequency $k$ can be represented by a three-dimensional vector U_1, U_2, U_3, U_4, or U_5. The invention operates by defining a binning function BF with respect to e.g. one channel C (usually more than one channel are investigated). The operation of the binning function BF is schematically indicated in Figure 7 by a doted arrow, and the five vectors U_1, U_2, U_3, U_4, and U_5 are schematically modified into U_1', U_2', U_3', U_4', and U_5', respectively.

In situation A, a binning function BF is applied on one frequency indicated by vector U_1 and as a result of the binning function BF, the Fourier coefficients Usk_X(k) of U_1 are modified and therefore the vector changed as shown.

In situation B, a binning function BF is applied on two frequencies indicated by vectors U_2 and U_3 and as a result of the binning function BF, the Fourier coefficients Usk_X(k) of both U_2 and U_3 are modified into vectors U_2' and U_3', respectively. I n this particular case, the binning function BF has the effect that U_2' is equal to U_3'. This may for example be the case of a binning function BF that significantly alters values e.g. a harsh rounding down or similar. Thus, information is lost but more easy and/or improved analysis may be performed.

In situation C, a binning function BF is applied on two frequencies indicated by vectors U_4 and U_5 and as a result of the binning function BF, the Fourier coefficients Usk_X(k) of both U_4 and U_5 are modified into vectors U_4' and U_5', respectively. I n this particular case, the binning function BF has the effect of turning, in the vector space, the two vectors U_4 and U_5.

Fig. 8 is a schematic drawing of the spectra at various frequencies according to the present invention listing specifically the Fourier coefficients Usk_X(k) for the different spectra 20 that are consecutively numbered downwards in the left part of the Figure by the running index *s*. Also the frequency *k* is indicated in the top part of Figure 8. The frequency of the DFT runs from 1 to the maximum frequency *km* of the Fourier transform. Like before the four nucleotide bases A, T, C, and G constitutes four channels i.e. X= A, T, C, and G. Usually more than one channel are investigated, and thereby the similarity with the search template can be based on the degree of variation in more than one channel, e.g. X= A and C, and particularly, the similarity can based on the degree of variation of all the channels i.e. X = A, T, C, and G. To emphasize that each entry in Figure 8 comprise 4 different channels the entry named U1k_x in the first row (s=1) has been blown up and all four channels written out explicitly in the upper part of Figure 8.

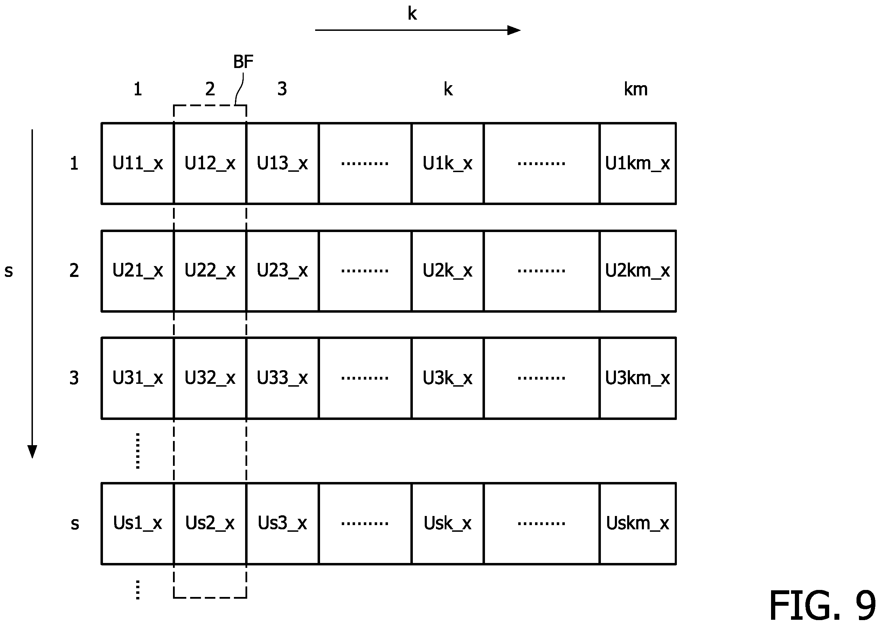

Fig. 9 is a drawing similar to Figure 8 showing a binning function BF according to the present invention. A plurality of spectra s is obtained based on the DNA sequence 10 by converting the DNA sequence into a plurality of binary indicator sequences (BIS), and applying short term Fourier transform (STFT) on the binary indicator sequences, each spectrum comprising corresponding frequencies k and Fourier coefficients Usk_X(k), where each kind of Fourier coefficient constitutes a channel X.

There is then defined a binning function BF for a frequency K', here K'=2, applicable to the Fourier coefficients Usk_X(k) with respect to the relevant channels X. Thus, the binning function can for example comprises truncation, rounding up, rounding down, a modular function, and/or a threshold function or other relevant mathematical function relevant for the purpose of the present invention. In one embodiment, a truncation is performed. Typically, the binning function (BF) is defined for all channels X, thus, X= {A, T, C, and G}, but for some application one or a subset, e.g. C and G, can be channels to be analyzed. In Figure 9, the binning function (BF) is applied on the portion of the plurality of spectra from s=1 to s, and thereby modifies the corresponding Fourier coefficients Usk_X(k). Alternatively, the binning function (BF) could is applied on a smaller portion, e.g. s=1 to s= 2.

There after substantially equal modified Fourier coefficients Usk_X(k) within the said portion of the plurality of spectra, e.g. s=1 and upwards, is found and preferably marked or tagged for further analysis. Thus by finding is meant for instance counting how many entries having a certain value of the modified Fourier coefficients Usk_X(k), e.g. 10. The term "substantially equal" is meant to take into account of numerical errors introduced after application of the binning function BF.

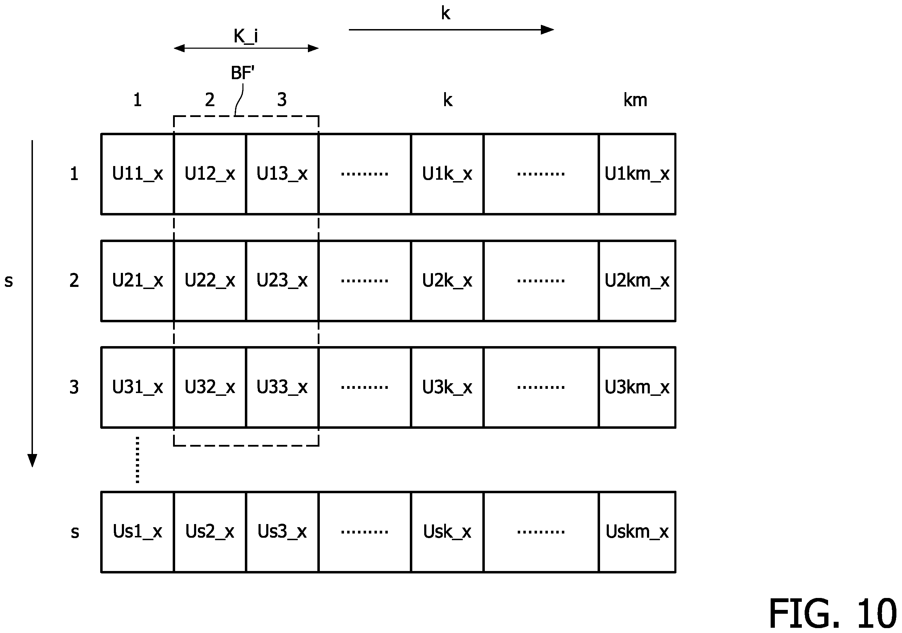

Fig. 10 is a drawing similar to Figure 8 showing another binning function BF' according to the present invention. The method may be repeated for a set of frequencies K_i., either in parallel or subsequently, typically in an interval but the set K_i could also "jump" over certain k values. It should therefore be emphasized that the frequency set or interval K_i can comprise several distinctive frequency intervals i.e. K_i may comprise k= 2, k= 6 or k=2 and k= 4. Thus, K_i can be any suitable subgroup or combination of subgroups within the interval from k=1 to k=km (the maximum frequency of the Fourier transform).

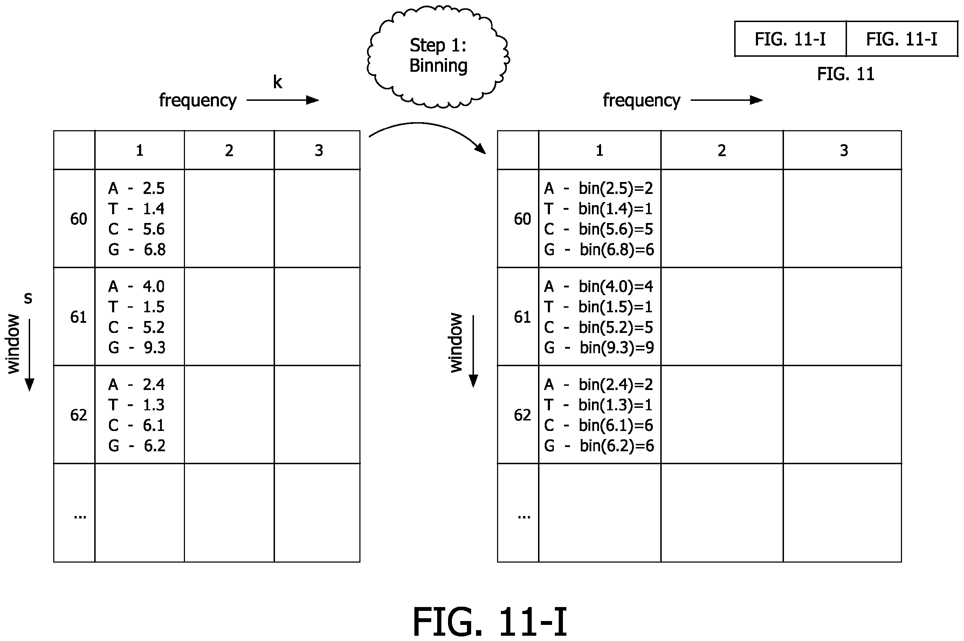

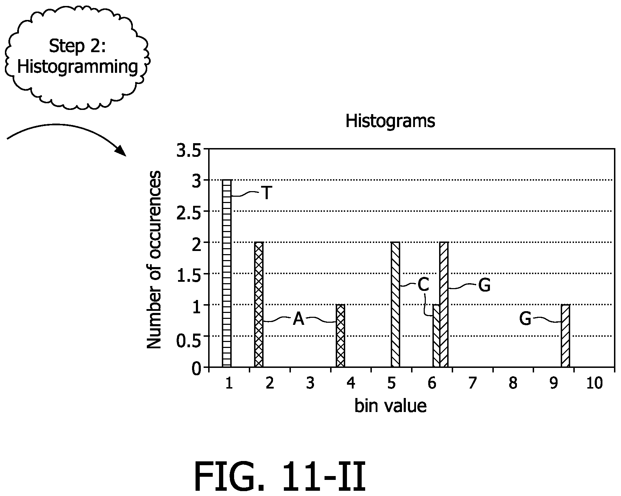

Fig. 11 is a drawing similar to Figure 8 schematically illustrating application of a binning function BF on a plurality of spectra but for simplicity only with one frequency *k*. After applying the binning function BF, in this case a simple truncation, the equal values of modified Fourier coefficients are found, the number of occurrence are then plotted into a histogram as a function of the binned value, e.g. two occurrences of Us1_G(k) = 6 and one occurrence of Us1_G(k) = 9 and so forth.

For each frequency, values that are "similar", i.e. substantially equal according to the applied binning function BF, are grouped together and histograms showing the number of values falling in each bin are built. The values of A, C, G, T for an individual frequency can be compared independently, or combined in a common measure taking into account similarities on all four nucleotides to find similarities in that frequency. Figure 11 provides an example of how the binning function BF is applied and histograms are generated. Afterwards various embodiments of frequency sorting or clustering methods may be applied. Using the binning function, histograms showing the "similar" values are generated for A, T, C and G for all frequencies.

Next, for each frequency one or more histogram bins (e.g. the largest) are selected, according to a chosen strategy. In the following three such strategies: Top Down Hierarchical Sorting (TDHS), Independent Iterative Sorting (IIS), and Lattice Sorting (LS), are further explained, but other methods are readily available to the skilled person within the context and teaching of the present invention. The domain may then be split according to the chosen strategy and taking into account the histogram bins, and the process is repeated in each of the sub-domains until a stopping criterion is reached.

For instance, when the largest bin is selected, it provides the largest number of sequences that share a "similar" value according to the binning function BF in that specific frequency for one of the nucleotides. The frequency for the largest value in all histogram bins across all frequencies (for each frequency there is a single histogram) is selected and the sequences contributing to that histogram are grouped together. The whole domain of sequences is split this way into the group of sequences sharing a similarity in that frequency and the rest, obtaining two "clusters" (although this is not a clustering algorithm in the strict sense of the word this terminology may be adopted), and a specific selection and processing strategy is applied on each of the two clusters. Next, histograms of the values are built again or the computed histograms bins are updated to reflect the split into clusters; the longest histogram is selected, and according to that histogram the domain is split into two clusters again. The iterations stop when the size of the longest histogram is below a predefined threshold, when the user-defined number of long patterns to be extracted was reached, or when each of the two clusters contains a single sequence. Other stop criterion may also be applied.

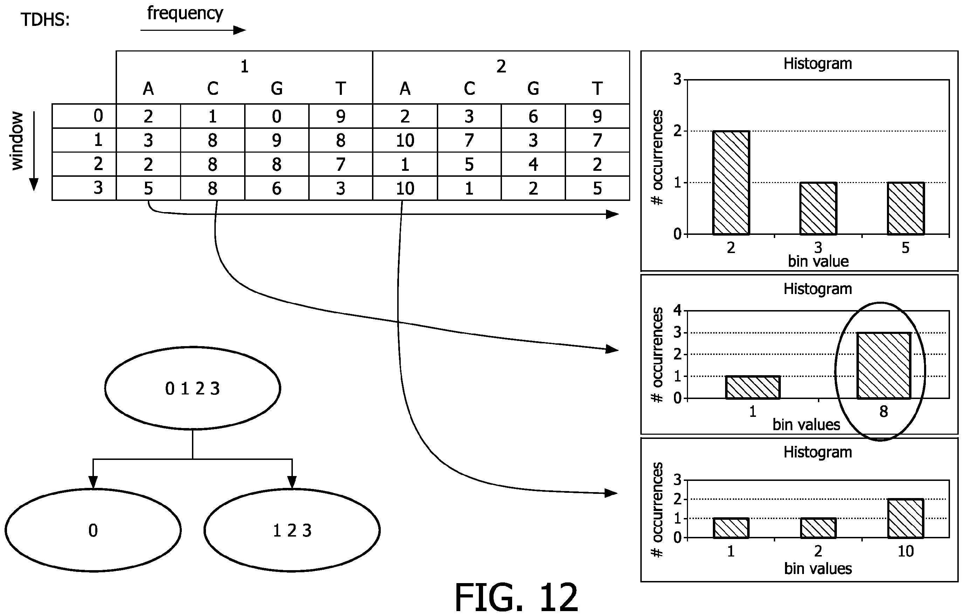

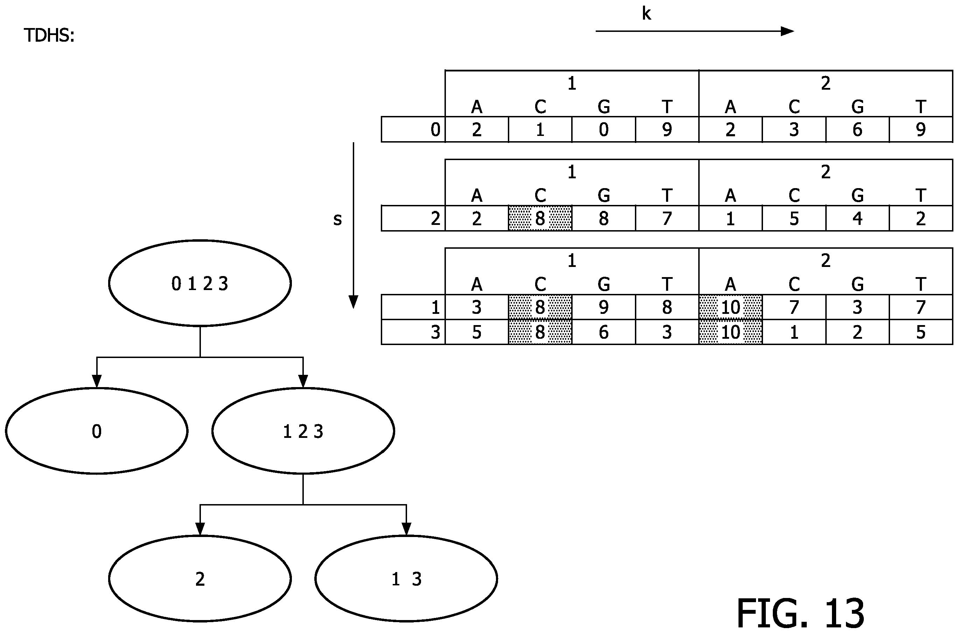

Fig. 12 and 13 show an example of a so-called top-down hierarchical sorting (TDHS) according to the present invention. Once the longest pattern is found, in example k=1, C channel, three times the value "8", the TDHS algorithm splits the domain of windows or spectra into those containing the longest pattern and the rest. To illustrate this process, the histogram of three selected channels are shown to the right, i.e. k=1, A & C channel, and k=2, A channel. With the solid circle in the middle histogram the longest pattern is identified schematically.

Next, in each of the two clusters, or the first and second group, the (next) longest pattern is found and the clusters are again each split or subdivided into clusters or groups containing the long pattern and the rest. This is illustrated in Figure 13, where windows or spectra s=1, 2 and 3 forms a group which is split into a group of spectra containing the longest pattern k=2, A channel with two times the binned value "10", and the group with spectra s=2.

This hierarchical sorting is illustrated by the "sorting three" to lower left in Figure 13 with two branching points. The first branching of the TDHS sorting is also shown in the lower left of Figure 12.

This algorithm stops when a threshold for the longest pattern or for the number of steps was reached, or when each of the two clusters or groups contains a single sequence, e.g. spectrum s=2 in Figure 13. In the end, one will have a hierarchy of patterns. One may choose to display at each step of separation both clusters, or only that cluster or group with the longest pattern. This strategy may miss long patterns when they are split in a previous step. One variation on TDHS is to stop splitting the left side of the tree - the one that already contains the longest pattern. This will result in a multi-leaved binary tree.

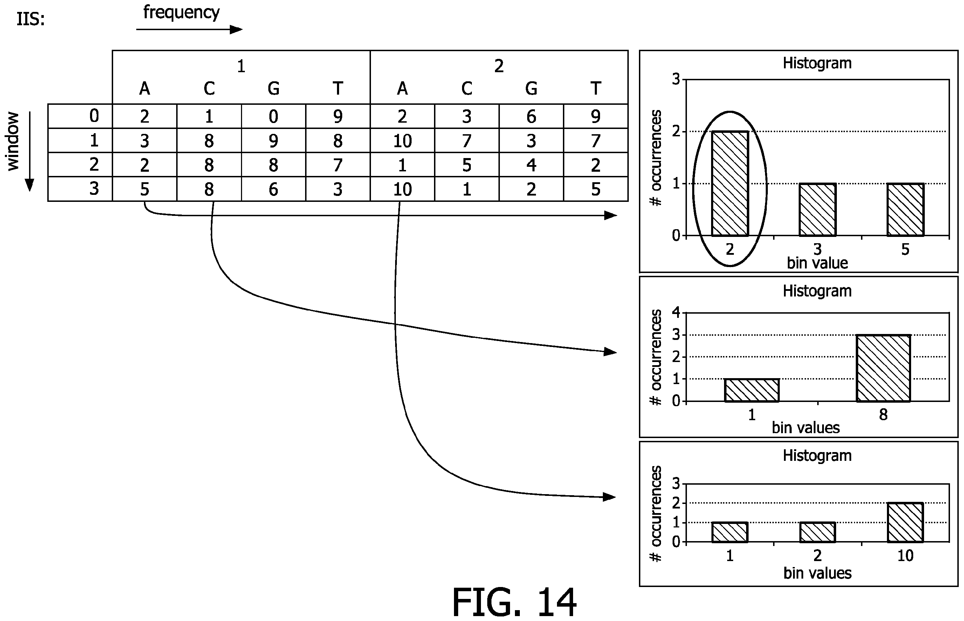

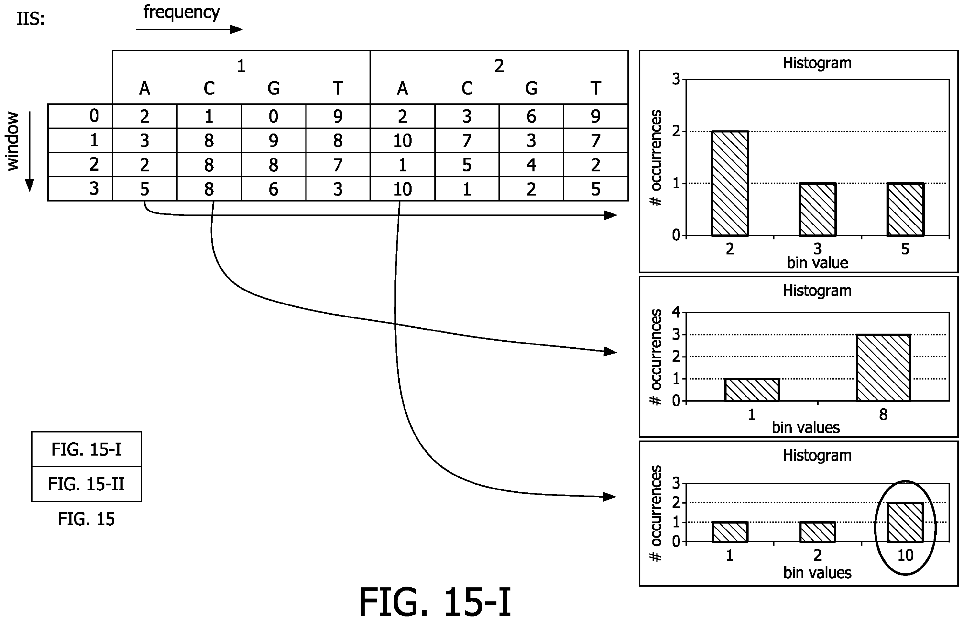

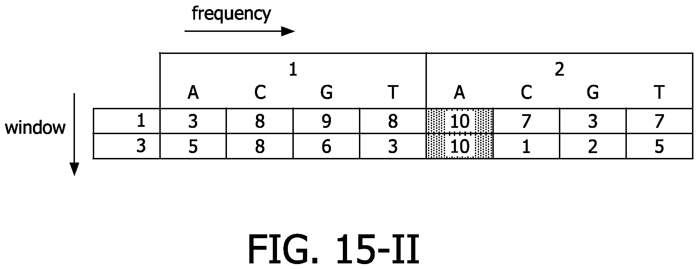

Fig. 14 and 15 show an example of a so-called independent iterative sorting (IIS) according to the present invention. IIS displays all the patterns in the domain in decreasing order of their size. It first selects the longest pattern as shown in Figure 12 for the TDHS sorting algorithm, then the IIS algorithm re-orders the cluster that contains the longest pattern on top and displays the entire domain. Next IIS selects the second (distinct) longest pattern independent of the first pattern as shown in Figure 14 with k=1, channel A having two occurrence of binned value "2", indicated in the histogram with the solid circle (though k=2, channel A also has two occurrences of binned value "10"), and so on until all patterns have been found. Thus, in Figure 15, the third longest pattern is k=2, channel A, with two occurrences of binned value "10", as also indicated in the histogram with a solid circle. With this strategy fully coexisting patterns (no gap in the longer pattern), or fully disjoint patterns (no common sequences) will always appear. It should also note that the clusters obtained in different iterations might contain same (overlapping) spectra.

Furthermore, a so-called lattice sorting (LS) algorithm may be implemented in connection with the present invention. Initially, for all patterns longer than a given size N_thresl (or alternatively for the k longest patterns) form clusters by selecting the rows or spectra including those patterns and discarding the rest. Afterwards, there is performed the same selection iteratively in each cluster or group until no suitable patterns are found, i.e. until all patterns are shorter than N_thres2 (or all patterns left are of length 1). With this strategy the clusters can be overlapping, and each cluster has one child. Unlike TDHS, LS never misses long patterns. Also with this strategy fully coexisting patterns will always appear.

All the above strategies of TDHS, IIS, and LS can implemented interactive, in the sense that at each step the patterns can be visualized and the user can decide which branches in the hierarchies of clusters or groups to explore.

Next, the spectra may be stacked on top of each other in a new representation called sorted video as shown in Figure 6, and displayed. Depending on the user's preference, all clusters can be shown, or only those that contain the strongest pattern in that algorithmic step.

In addition, this present invention is conducive to parallelization unlike other clustering methods known in the art (say hierarchical clustering). For sorting, the histograms are built for each frequency, which makes it easy to split the domain of Fourier values among several processes and execute them in parallel, on a parallel or distributed system, or on grids.

At the end, the present invention provides a visualization method (as in Figure 6), which makes it easier for the biologist or clinician to see the results and find further explanation about the similarity of these patterns. For this task, the genomic annotation available, say name of gene, or genomic element, species, experiment, etc., may be provided.

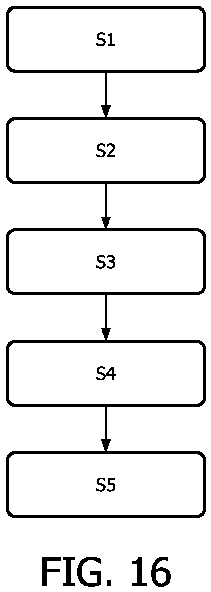

Fig. 16 is a flow chart of a method according to the invention. The method comprises:

**S1** providing a DNA sequence,

**S2** creating a plurality of spectra 20 based on the DNA sequence by converting the DNA sequence into a plurality of binary indicator sequences (BIS), and applying short term Fourier transform (STFT) on the binary indicator sequences, each spectrum comprising corresponding frequencies k and Fourier coefficients Usk_X(k), where each kind of Fourier coefficient constitutes a channel X,

**S3** defining a binning function BF for a frequency K' applicable to the Fourier coefficients Usk_X(k) with respect to one or more channels X,

**S4** applying the binning function BF on at least a portion of the plurality of spectra and thereby modifying the corresponding Fourier coefficients Usk_X(k), and

**S5** finding substantially equal modified Fourier coefficients Usk_X(k) within the said portion of the plurality of spectra.

The invention can be implemented in any suitable form including hardware, software, firmware or any combination of these. The invention or some features of the invention can be implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described in connection with the specified embodiments, it is not intended to be limited to the specific form set forth herein. Rather, the scope of the present invention is limited only by the accompanying claims. In the claims, the term "comprising" does not exclude the presence of other elements or steps. Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

**Claims**

1. A method for identifying structurally or compositionally similar patterns of a DNA sequence (10) that exhibit similar spectral properties, the method comprising:

   providing a DNA sequence,
   creating a plurality of spectra (20) based on the DNA sequence by converting the DNA sequence into a plurality of binary indicator sequences (BIS), and applying short term Fourier transform (STFT) on the binary indicator sequences, each spectrum comprising corresponding frequencies (k) and Fourier coefficients (Usk_X(k)), where each kind of Fourier coefficient constitutes a channel (X),
   defining a binning function (BF) for a frequency (K') applicable to the Fourier coefficients (Usk_X(k)) with respect to one or more channels (X),
   applying the binning function (BF) on at least a portion of the plurality of spectra and thereby modifying the corresponding Fourier coefficients (Usk_X(k)), and
   finding substantially equal modified Fourier coefficients (Usk_X(k)) within the said portion of the plurality of spectra, and
   wherein a first group of spectra (S) with the largest set of substantially equal modified Fourier coefficients (Usk_X(k)) in any frequency and/or channel is found and separated from the remaining spectra, the remaining spectra forming a second group of spectra.

2. The method according to claim 1, wherein the finding of substantially equal modified Fourier coefficients (Usk_X(k)) within the said portion of the plurality of spectra comprises a quantitative analysis of a distribution of the modified Fourier coefficients (Usk_X(k)) with respect to the said binning function (BF).

3. The method according to claim 1, wherein the method is repeated for a set of frequencies (K_i).

4. The method according claim 1, wherein the set of binary indicator sequences is reduced into a smaller set of BIS using a merge function, the merge function preferably comprising a logical AND function.

5. The method according to claim 1, wherein the largest set of substantially equal modified Fourier coefficients (Usk_X(k)) is found and separated within the second group of spectra.

6. The method according to claim 5, wherein the separation of spectra into a first and a second group of spectra is repeated disregarding the previously found longest set of modified Fourier coefficients (Usk_X(k)).

7. The method according to claim 5 or 6, wherein the separation of spectra into a first and a second group is repeated i) until a pre-defined threshold for the longest set of modified Fourier coefficients (Usk_X(k)) is found, ii) until a pre-defined number of separations into a first and a second group of spectra is performed, or iii) until the first and/or the second group of spectra contains a single sequence

8. The method according to claim 1 or 3, wherein a first group of spectra (S) with the largest set of substantially equal modified Fourier coefficients (Usk_X(k)) in any frequency and/or channel is found and marked.

9. The method according to claim 8, wherein a second group of spectra with the largest set of substantially equal modified Fourier coefficients (Usk_X(k)) in any frequency and/or channel is found, disgarding the previously found longest set of modified Fourier coefficients (Usk_X(k)), and marked.

10. The method according to any of claims 8-9, wherein the longest set is found and the group of spectra is reordered i) until a pre-defined threshold for the length of longest set of the modified Fourier coefficients (Usk_X(k)) is found, ii) until a pre-defined number of longest set is found, or iii) until the longest set contains a single sequence.

**11.** The method according to claim 1 or 3, wherein all groups of spectra (S) having length of a pattern of found modified Fourier coefficients (Usk_X(k)) being above a first predefined threshold (N_thres1), or all groups of spectra containing the **k** longest patterns, **k** being an integer, are found and separated from the remaining spectra, the remaining spectra forming a second group of spectra.

**12.** The method according to claim 11, wherein each group of spectra separated according to claim 11 is further separated using a second predefined threshold (N_thres2) for the length of the pattern of modified Fourier coefficients (Usk_X(k)), or using the j longest patterns, **j** being an integer equal or different from **k.**

**13.** The method according to claim 12, wherein the separation of spectra into groups is repeated i) until a pre-defined threshold for length of the patterns of modified Fourier coefficients (Usk_X(k)) is found, ii) until a pre-defined number of separations into a first and a second group of spectra is performed, or iii) until the first and/or the second group of spectra contains sequences of modified Fourier coefficients (Usk_X(k)) having length equal to one.

**14.** A computer program product comprising instructions which, when the program is executed by a computer, cause the computer to carry out the method of claim 1.

**Patentansprüche**

**1.** Verfahren zum Identifizieren von strukturell oder kompositionell ähnlichen Mustern einer DNA-Sequenz (10), die ähnliche spektrale Eigenschaften aufweisen, wobei das Verfahren Folgendes umfasst:

Bereitstellen einer DNA-Sequenz;
Erstellen einer Vielzahl von auf der DNA-Sequenz basierenden Spektren (20) durch Umwandeln der DNA-Sequenz in eine Vielzahl von binären Indikatorsequenzen (BIS) und Anwenden einer Short-Term-Fourier-Transformation (STFT) auf die binären Indikatorsequenzen, wobei jedes Spektrum entsprechende Frequenzen (k) und Fourier-Koeffizienten (Usk_X(k)) umfasst, wobei jede Art von Fourier-Koeffizient einen Kanal (X) darstellt,
Definieren einer Binning-Funktion (BF) für eine Frequenz (K'), die auf die Fourier-Koeffizienten (Usk_X(k)) in Bezug auf einen oder mehrere Kanäle (X) anwendbar ist,
Anwenden der Binning-Funktion (BF) auf mindestens einen Teil der Vielzahl von Spektren und dadurch Modifizieren der entsprechenden Fourier-Koeffizienten (Usk_X(k)), und
Finden von im Wesentlichen gleichen modifizierten Fourier-Koeffizienten (Usk_X(k)) innerhalb des genannten Teils der Vielzahl von Spektren, und
wobei eine erste Gruppe von Spektren (S) mit dem größten Satz an im Wesentlichen gleichen modifizierten Fourier-Koeffizienten (Usk_X(k)) in irgendeiner Frequenz und/oder irgendeinem Kanal gefunden wird und von den verbleibenden Spektren separiert wird, wobei die verbleibenden Spektren eine zweite Gruppe von Spektren bilden.

**2.** Verfahren nach Anspruch 1, wobei das Finden von im Wesentlichen gleichen modifizierten Fourier-Koeffizienten (Usk_X(k)) innerhalb des genannten Teils der Vielzahl von Spektren eine quantitative Analyse einer Verteilung der modifizierten Fourier-Koeffizienten (Usk_X(k)) in Bezug auf die genannte Binning-Funktion (BF) umfasst.

**3.** Verfahren nach Anspruch 1, wobei das Verfahren für einen Satz von Frequenzen (K_i) wiederholt wird.

**4.** Verfahren nach Anspruch 1, wobei der Satz von binären Indikatorsequenzen mithilfe einer Merge-Funktion zu einem kleineren Satz von BIS reduziert wird, wobei die Merge-Funktion vorzugsweise eine logische UND-Funktion umfasst.

**5.** Verfahren nach Anspruch 1, wobei der größte Satz von im Wesentlichen gleichen modifizierten Fourier-Koeffizienten (Usk_X(k)) innerhalb der zweiten Gruppe von Spektren gefunden und separiert wird.

**6.** Verfahren nach Anspruch 5, wobei das Separieren von Spektren in eine erste Gruppe und eine zweite Gruppe von Spektren unabhängig von dem zuvor gefundenen längsten Satz von modifizierten Fourier-Koeffizienten (Usk_X(k)) wiederholt wird.

**7.** Verfahren nach Anspruch 5 oder 6, wobei das Separieren von Spektren in eine erste und eine zweite Gruppe wiederholt wird, i) bis ein vordefinierter Schwellenwert für den längsten Satz von modifizierten Fourier-Koeffizienten (Usk_X(k)) gefunden wurde, (ii) bis eine vordefinierte Anzahl von Separierungen in eine erste und eine zweite Gruppe von Spektren durchgeführt wurde, oder iii) bis die erste und/oder die zweite Gruppe von Spektren eine einzelne Sequenz enthält.

8.  Verfahren nach Anspruch 1 oder 3, wobei eine erste Gruppe von Spektren (S) mit dem größten Satz von im Wesentlichen gleichen modifizierten Fourier-Koeffizienten (Usk_X(k)) in irgendeiner Frequenz und/oder irgendeinem Kanal gefunden und markiert wird.

9.  Verfahren nach Anspruch 8, wobei eine zweite Gruppe von Spektren mit dem größten Satz von im Wesentlichen gleichen modifizierten Fourier-Koeffizienten (Usk_X(k)) in irgendeiner Frequenz und/oder irgendeinem Kanal gefunden wird, unabhängig von dem zuvor gefundenen längsten Satz von modifizierten Fourier-Koeffizienten (Usk_X(k)), und markiert wird.

10. Verfahren nach einem der Ansprüche 8 bis 9, wobei der längste Satz gefunden wird und die Gruppe von Spektren neu geordnet wird, i) bis ein vordefinierter Schwellenwert für die Länge des längsten Satzes der modifizierten Fourier-Koeffizienten (Usk_X(k)) gefunden wurde, ii) bis eine vordefinierte Anzahl von längsten Satz gefunden wurde oder iii) bis der längste Satz eine einzelne Sequenz enthält.

11. Verfahren nach Anspruch 1 oder 3, wobei alle Gruppen von Spektren (S) mit einer Länge eines Musters von gefundenen modifizierten Fourier-Koeffizienten (Usk_X(k)), die über einem ersten vordefinierten Schwellenwert (N_thres1) liegt, oder alle Gruppen von Spektren, die die k längsten Muster enthalten, wobei k eine ganze Zahl ist, gefunden werden und von den verbleibenden Spektren separiert werden, wobei die verbleibenden Spektren eine zweite Gruppe von Spektren bilden.

12. Verfahren nach Anspruch 11, wobei jede Gruppe von Spektren, die nach Anspruch 11 separiert wurde, unter Verwendung eines zweiten vordefinierten Schwellenwerts (N_thres2) für die Länge des Musters von modifizierten Fourier-Koeffizienten (Usk_X(k)) oder unter Verwendung der j längsten Muster, wobei j eine ganze Zahl gleich oder verschieden von k ist, weiter separiert wird.

13. Verfahren nach Anspruch 12, wobei das Separieren von Spektren in Gruppen wiederholt wird, i) bis ein vordefinierter Schwellenwert für die Länge der Muster von modifizierten Fourier-Koeffizienten (Usk_X(k)) gefunden wurde, ii) bis eine vordefinierte Anzahl von Separierungen in eine erste und eine zweite Gruppe von Spektren durchgeführt wurde, oder iii) bis die erste und/oder die zweite Gruppe von Spektren Sequenzen von modifizierten Fourier-Koeffizienten (Usk_X(k)) mit einer Länge gleich eins enthält.

14. Computerprogrammprodukt umfassend Anweisungen, die, wenn das Programm durch einen Computer ausgeführt wird, den Computer veranlassen, das Verfahren nach Anspruch 1 durchzuführen.


**Revendications**

1.  Procédé pour identifier la structure ou la composition de profils similaires d'une séquence d'ADN (10) qui présentent des propriétés spectrales similaires, le procédé comprenant :

    la fourniture d'une séquence d'ADN,
    la création d'une pluralité de spectres (20) sur la base de la séquence d'ADN par la conversion de la séquence d'ADN en une pluralité de séquences d'indicateur binaire (BIS), et l'application d'une transformée de Fourier à court terme (STFT) aux séquences d'indicateur binaire, chaque spectre comprenant des fréquences (k) et coefficients de Fourier (Usk_X(k)) correspondants, où chaque type de coefficient de Fourier constitue un canal (X),
    la définition d'une fonction de répartition (BF) pour une fréquence (K') applicable aux coefficients de Fourier (Usk_X(k)) par rapport à un ou plusieurs canaux (X),
    l'application de la fonction de répartition (BF) à au moins une partie de la pluralité de spectres et ainsi la modification des coefficients de Fourier (Usk_X(k)) correspondants, et
    le fait de trouver des coefficients de Fourier (Usk_X(k)) modifiés sensiblement égaux dans ladite partie de la pluralité de spectres, et
    dans lequel un premier groupe de spectres (S) ayant le plus grand ensemble de coefficients de Fourier (Usk_X(k)) modifiés sensiblement égaux dans n'importe quelle fréquence et/ou n'importe quel canal est trouvé et séparé des spectres restants, les spectres restants formant un seconde groupe de spectres.

**2.** Procédé selon la revendication 1, dans lequel le fait de trouver des coefficients de Fourier (Usk_X(k)) modifiés sensiblement égaux dans ladite partie de la pluralité de spectres comprend une analyse quantitative d'une distribution des coefficients de Fourier (Usk_X(k)) modifiés par rapport à ladite fonction de répartition (BF).

**3.** Procédé selon la revendication 1, dans lequel le procédé est répété pour un ensemble de fréquences (K_i).

**4.** Procédé selon la revendication 1, dans lequel l'ensemble de séquences d'indicateur binaire est réduit pour obtenir un plus petit ensemble de BIS en utilisant une fonction de fusion, la fonction de fusion comprenant de préférence une fonction ET logique.

**5.** Procédé selon la revendication 1, dans lequel le plus grand ensemble de coefficients de Fourier (Usk_X(k)) modifiés sensiblement égaux est trouvé et séparé au sein du second groupe de spectres.

**6.** Procédé selon la revendication 5, dans lequel la séparation des spectres en un premier et un second groupe de spectres est répétée sans prendre en compte l'ensemble le plus long précédemment trouvé de coefficients de Fourier (Usk_X(k)) modifiés.

**7.** Procédé selon la revendication 5 ou 6, dans lequel la séparation des spectres en un premier et un second groupe est répétée i) jusqu'à ce qu'un seuil prédéfini pour l'ensemble le plus long de coefficients de Fourier (Usk_X(k)) modifiés soit trouvé, ii) jusqu'à ce qu'un nombre prédéfini de séparations en un premier et un second groupe de spectres soit réalisé, ou iii) jusqu'à ce que le premier et/ou le second groupe de spectres contiennent une unique séquence.

**8.** Procédé selon la revendication 1 ou 3, dans lequel le premier groupe de spectres (S) ayant le plus grand ensemble de coefficients de Fourier (Usk_X(k)) modifiés sensiblement égaux dans n'importe quelle fréquence et/ou n'importe quel canal est trouvé et marqué.

**9.** Procédé selon la revendication 8, dans lequel un second groupe de spectres ayant le plus grand ensemble de coefficients de Fourier (Usk_X(k)) modifiés sensiblement égaux dans n'importe quelle fréquence et/ou n'importe quel canal est trouvé, sans prendre en compte l'ensemble le plus long précédemment trouvé de coefficients de Fourier (Usk_X(k)) modifiés, et marqué.

**10.** Procédé selon l'une quelconque des revendications 8 et 9, dans lequel l'ensemble le plus long est trouvé et le groupe de spectres est réorganisé i) jusqu'à ce qu'un seuil prédéfini pour la longueur de l'ensemble le plus long de coefficients de Fourier (Usk_X(k)) modifiés soit trouvé, ii) jusqu'à ce qu'un nombre prédéfini d'ensemble le plus long soit trouvé, ou iii) jusqu'à ce que l'ensemble le plus long contienne une unique séquence.

**11.** Procédé selon la revendication 1 ou 3, dans lequel tous les groupes de spectres (S) ayant une longueur d'un profil de coefficients de Fourier (Usk_X(k)) modifiés trouvés qui est au-dessus d'un premier seuil prédéfini (N_thres1), ou tous les groupes de spectres contenant les k profils les plus longs, k étant un nombre entier, sont trouvés et séparés des spectres restants, les spectres restants formant un second groupe de spectres.

**12.** Procédé selon la revendication 11, dans lequel chaque groupe de spectres séparé selon la revendication 11 est en outre séparé en utilisant un second seuil prédéfini (N_thres2) pour la longueur du profil de coefficients de Fourier (Usk_X(k)) modifiés, ou en utilisant les j profils les plus longs, j étant un nombre entier égal à k ou différent de k.

**13.** Procédé selon la revendication 12, dans lequel la séparation des spectres en groupes est répétée i) jusqu'à ce qu'un seuil prédéfini pour la longueur des profils de coefficients de Fourier (Usk_X(k)) modifiés soit trouvé, ii) jusqu'à ce qu'un nombre prédéfini de séparations en un premier et un second groupe de spectres soit réalisé, ou iii) jusqu'à ce que le premier et/ou le second groupe de spectres contiennent des séquences de coefficients de Fourier (Usk_X(k)) modifiés ayant une longueur égale à un.

**14.** Produit de programme informatique comprenant des instructions qui, quand le programme est exécuté par un ordinateur, amène l'ordinateur à mettre en oeuvre le procédé selon la revendication 1.

10

AACTGGCATCCGGGAATAAGGTCT ...

$u_A[n] = 11000001000000110110000 ...$

$u_T[n] = 00010000100000010000101 ...$

$u_C[n] = 00100010011000000000010 ...$

$u_G[n] = 00001100000111000011000 ...$

# FIG. 1

# FIG. 2

FIG. 3

$Su_A[k]$    x    $(R_A, G_A, B_A)$

$Su_T[k]$    x    $(R_T, G_T, B_T)$

$Su_C[k]$    x    $(R_C, G_C, B_C)$

$Su_G[k]$    x    $(R_G, G_G, B_G)$

FIG. 4

FIG. 5

FIG. 6

FIG. 7A

FIG. 7B

FIG. 7C

EP 2 310 968 B1

k →

|      | 1 | 2 | 3 | | k | | |
|------|---|---|---|---|---|---|---|
| 1 | U11_x | U12_x | U13_x | ········ | U1k_x | ········ | U1km_x |

U1k_A
U1k_T
U1k_C
U1k_G

~20

s ↓

| 2 | U21_x | U22_x | U23_x | ········ | U2k_x | ········ | U2km_x | ~20 |

| 3 | U31_x | U32_x | U33_x | ········ | U3k_x | ········ | U3km_x | ~20 |

| s | Us1_x | Us2_x | Us3_x | ········ | Usk_x | ········ | Uskm_x |

FIG. 8

FIG. 9

EP 2 310 968 B1

k

K_i

BF'

| | 1 | 2 | 3 | | k | | km |
|---|---|---|---|---|---|---|---|
| 1 | U11_x | U12_x | U13_x | ········ | U1k_x | ········ | U1km_x |
| 2 | U21_x | U22_x | U23_x | ········ | U2k_x | ········ | U2km_x |
| 3 | U31_x | U32_x | U33_x | ········ | U3k_x | ········ | U3km_x |
| s | Us1_x | Us2_x | Us3_x | ········ | Usk_x | ········ | Uskm_x |

s

FIG. 10

Step 1:
Binning

| FIG. 11-I | FIG. 11-I |

FIG. 11

frequency →  k

window ↓ s

| | 1 | 2 | 3 |
|---|---|---|---|
| 60 | A - 2.5<br>T - 1.4<br>C - 5.6<br>G - 6.8 | | |
| 61 | A - 4.0<br>T - 1.5<br>C - 5.2<br>G - 9.3 | | |
| 62 | A - 2.4<br>T - 1.3<br>C - 6.1<br>G - 6.2 | | |
| ... | | | |

frequency →

window ↓

| | 1 | 2 | 3 |
|---|---|---|---|
| 60 | A - bin(2.5)=2<br>T - bin(1.4)=1<br>C - bin(5.6)=5<br>G - bin(6.8)=6 | | |
| 61 | A - bin(4.0)=4<br>T - bin(1.5)=1<br>C - bin(5.2)=5<br>G - bin(9.3)=9 | | |
| 62 | A - bin(2.4)=2<br>T - bin(1.3)=1<br>C - bin(6.1)=6<br>G - bin(6.2)=6 | | |
| ... | | | |

FIG. 11-I

FIG. 11-II

EP 2 310 968 B1

FIG. 12

TDHS:

$k \longrightarrow$

|   | 1 | | | | 2 | | | |
|---|---|---|---|---|---|---|---|---|
|   | A | C | G | T | A | C | G | T |
| 0 | 2 | 1 | 0 | 9 | 2 | 3 | 6 | 9 |

|   | 1 | | | | 2 | | | |
|---|---|---|---|---|---|---|---|---|
|   | A | C | G | T | A | C | G | T |
| 2 | 2 | 8 | 8 | 7 | 1 | 5 | 4 | 2 |

|   | 1 | | | | 2 | | | |
|---|---|---|---|---|---|---|---|---|
|   | A | C | G | T | A | C | G | T |
| 1 | 3 | 8 | 9 | 8 | 10 | 7 | 3 | 7 |
| 3 | 5 | 8 | 6 | 3 | 10 | 1 | 2 | 5 |

$s$

Tree:

0 1 2 3
→ 0
→ 1 2 3
  → 2
  → 1 3

FIG. 13

EP 2 310 968 B1

IIS:

frequency →

| | | 1 | | | | 2 | | |
|---|---|---|---|---|---|---|---|---|
| | A | C | G | T | A | C | G | T |
| 0 | 2 | 1 | 0 | 9 | 2 | 3 | 6 | 9 |
| 1 | 3 | 8 | 9 | 8 | 10 | 7 | 3 | 7 |
| 2 | 2 | 8 | 8 | 7 | 1 | 5 | 4 | 2 |
| 3 | 5 | 8 | 6 | 3 | 10 | 1 | 2 | 5 |

window ↓

FIG. 14

FIG. 15-I

EP 2 310 968 B1

EP 2 310 968 B1

frequency →

window ↓

| | 1 | | | | 2 | | | |
|---|---|---|---|---|---|---|---|---|
| | A | C | G | T | A | C | G | T |
| 1 | 3 | 8 | 9 | 8 | 10 | 7 | 3 | 7 |
| 3 | 5 | 8 | 6 | 3 | 10 | 1 | 2 | 5 |

# FIG. 15-II

28

```
┌─────────────────┐
│       S1        │
└─────────────────┘
         │
         ▼
┌─────────────────┐
│       S2        │
└─────────────────┘
         │
         ▼
┌─────────────────┐
│       S3        │
└─────────────────┘
         │
         ▼
┌─────────────────┐
│       S4        │
└─────────────────┘
         │
         ▼
┌─────────────────┐
│       S5        │
└─────────────────┘
```

# FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6287773 B
- PH 008112

**Non-patent literature cited in the description**

- **BENSON et al.** *Nucleic Acid Research,* vol. 18 (21), 6305-6310
- *NUCLEIC ACID RESEARCH,* 1990, vol. 18 (10), 3001-3006
- **N. DIMITROVA et al.** Analysis and visualization of DNA spectrograms: open possibilities for genome research. *ACM MM.,* October 2006